Europäisches Patentamt

(19) European Patent Office

Office européen des brevets

(11) Publication number: **0 276 984**
A2

# (12) EUROPEAN PATENT APPLICATION

(21) Application number: 88300640.5

(22) Date of filing: 26.01.88

(51) Int. Cl.⁴: **G 01 N 33/53**
G 01 N 33/564, C 12 Q 1/68
// C12N15/00

(30) Priority: 30.01.87 JP 19970/87

(43) Date of publication of application:
03.08.88 Bulletin 88/31

(84) Designated Contracting States:
AT BE CH DE ES FR GB GR IT LI LU NL SE

(71) Applicant: **DIAGNOSTIC PRODUCTS CORPORATION**
**5700 West 96th Street**
**Los Angeles California 90045 (US)**

(72) Inventor: **Kawamura, Masahide**
**4-4 Tatsumidaia Ichitara-shi**
**Chiba-ken 290 (JP)**

(74) Representative: **Myerscough, Philip Boyd et al**
**J.A.Kemp & Co. 14, South Square Gray's Inn**
**London, WC1R 5EU (GB)**

(54) **Measuring anti-DNA antibodies.**

(57) A method and assay kit for measuring anti-DNA antibody titres in biological fluids by an immunological method using a dsDNA label free of ssDNA fragments, wherein the dsDNA label is prepared by recombinant DNA techniques and having a constant length of a well defined kbp and a given specific activity and that such labeled dsDNA be used as the tracer in said immunological method.

EP 0 276 984 A2

**Description**

## MEASURING ANTI-DNA ANTIBODIES

Serafrom patients suffering from systemic lupus erythematosus (SLE) contain autoantibodies which may react with a variety of nuclear components including nucleic acids, nuclear proteins and varying combinations of double stranded DNA (dsDNA) and single stranded DNA (ssDNA). Some react with individual bases or nucleotides, with guanine and oligo(dT) sequences and even synthetic polynucleotides. Schwartz, R. S. and Stollar, D., J. Clin. Invest. 75: 321 (1985). Isenberg, D. A., Madaio, M. P., Reichlin, M., Shoenfeld, Y., Ranch, J., Stollar, D. and Schwartz, R. S., Lancet, August 25, 1984. In addition, these autoantibodies react to complex lipidantigens such as cardiolipin and other platelet membrane phospholipids, antipoly(ADP-ribose), histones and with proteoglycans; namely, hyaluronic acid and chrondroitin sulfate. However, the reaction of antibodies with dsDNA or native DNA (nDNA) is the major serological marker of this disease and the ability to measure dsDNA has proved particularly useful in diagnosing and monitoring disease activity.

Many assay methods have been devised to detect and semi-quantitate dsDNA in SLE patients. These methods used to identify these autoantibodies have included gel diffusion, Seligmann, M., C. R. Acad. Sci. (Paris), 245: 243 (1957); complement fixation; Robbins, W. C., Holman, H. R., Deicher, H. and Kunkel, H. G., Proc. Soc. exp. Biol. (NY), 96: 575 (1957); passive cutaneous anaphylaxis; Deicher, H. R. G., Holman, H. R., Kunkel, H. G. and Ovary, Z., J. Immunol. 84: 106 (1960); bentonite aggulutination; Bozicevich, J., Nason, J. P. and Kayhoe, D. E., Proc. Soc. Exp. Biol. (NY), 103: 636 (1960); haemaglutination; Jokinen, E. J. and Julkunen, H., Ann. rheum. Dis. 24: 477 (1965); radioelectrophoresis; Bickel, Y. B., Barnett, E. V. and Pearson, C. M., Clin. exp. Immunol. 3: 641 (1968); radioassays using 3H or 125I; Wold, R. T., Young, F. E., Tan, E. M. and Farr, R. S., Science 161: 806 (1968); and enzyme immunoassays; Rubin, R. L., Joslin, F. G. and Tan, E. M., J. Immunol. Methods 63: 359 (1983). Most, if not all, of the aforementioned methods lack specificity and sensitivity. In most cases false positive results are reported due to lack of specificity for the differential diagnosis of SLE from other connective tissue diseases.

Radioassays and enzymeimmunoassays have been shown to give acceptable correlation with the degree and gravity of SLE conditions. However, these immunological methods all use labeled preparations of nDNA from calf thymus that is highly polymerized with high molecular mass DNA fragments of varying length, on the average, about 20 kilo base pair. Also, these preparations contain varying amounts of ssDNA.

These 20 kbp DNA molecules often bind nonspecifically to circulating plasma proteins, while the ssDNA contaminant in these preparations bind with complement Clq. Thus, these conventional immunological methods for measuring antibodies to dsDNA (nDNA) usually require pretreatment of serum or plasma sample at 56C for 30 minutes to inactive complement Clq prior to the immunological reaction between the DNA tracer and the circulating anti-DNA antibodies.

Under ideal conditions it is preferable that one molecule of circulating anti-DNA IgG molecule bind only one or two labeled dsDNA molecule(s) of a given length and free of contaminants, particularly ssDNA.

To this end in vivo techniques have been devised to prepare labeled dsDNA (especially 125I labeled) by introducing 125I-deoxycytidine into HeLa cells. However, these in vivo labeling methods do not produce high purity labeled dsDNA because of ssDNA contaminants are usually found in such preparations as by-products.

According to this invention, circulating anti-DNA antibodies in biological fluids are measured, for example, by a radioassay using a well defined 125-I-dsDNA tracer of a given kilobase-pair and prepared by recombinant DNA techniques and is devoid of any ssDNA contaminant. Plasmid for the in vitro preparation of such tracer and assay parameters and kit for measuring anti-DNA antibodies are hereinafter disclosed.

## SUMMARY OF THE INVENTION

Briefly, the present invention comrpises a method for measuring anti-DNA antibody titres in biological fluids by an immunological method using a dsDNA label free of ssDNA fragments, wherein the dsDNA label is prepared by recombinant DNA techniques and having a constant length of a well defined kbp and a given specific activity and that such labeled dsDNA be used as the tracer in said immunological method.

The invention also comprehends an assay kit for measuring anti-DNA antibody titres in biological fluids by an immunological method using dsDNA label free of ssDNA fragments, wherein the dsDNA label is prepared by recombinant DNA techniques and having a constant length of a well defined kbp and a given specific activity and that such labeled dsDNA be used as the tracer in said immunological method.

It is an object of the present invention is to circumvent the aforementioned shortcomings in immunological methods and in particular radioassays with 125I-labeles dsDNA and other labeled dsDNA assays.

In general, it is an object of this invention to provide a novel method of measuring anti-DNA titres in biological fluids.

It is also a general objective of this invention to provide a novel assay kit for carrying out the previously discussed measurements in biological fluids.

These and other objectives and advantages of this invention will be apparent from the more detailed discussion which follows, taken together with the accompanying drawings.

## DETAILED DESCRIPTION OF THE INVENTION

The object of the present invention is to devise a well defined base pair of a dsDNA molecule of a given length and that such preparation be free of ssDNA. Such preparation when labeled with a signal producing proble (125-iodine, enzyme label, fluorescent label or achemiluminescent or bioluminescent label) will have a well defined specific activity, is stable and will specifically bind to circulating anti-DNA antibodies in SLE serum or plasma.

To achieve such an objective a plasmid DNA had to be constructed and was prepared from Escherichiacoli which contains no ssDNA. This plasmid was cut with a suitable restriction enzyme to produce dsDNA of a given length not to exceed 1.5 kbp to be used as tracer material. The present plasmid used in the present disclosed invention is prepared from E. coli but it is possible to use any other suitable plasmid containing organisms such as Bacillussubtilius, Pseudomas putida, and Saccharamyces cereviciae. Other plasmid containing organisms will be obvious to those skilled in the art. In addition, chemically synthesized dsDNA may also be used to fulfill the objectives of this invention.

This invention will be described in detail with reference to the following examples which are presented for purposes of illustration only.

## EXAMPLE 1

### Preparation of plasmid DNA, pNDPC 1

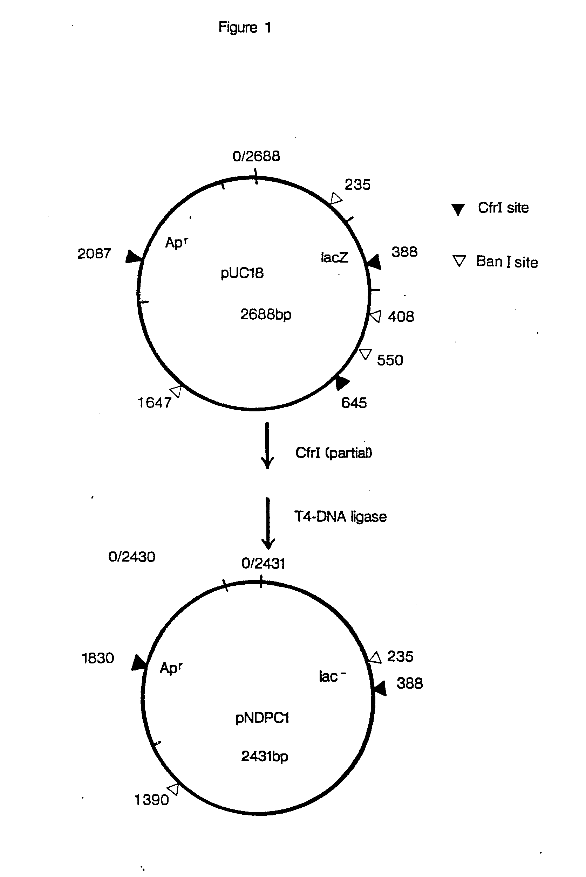

Figure 1 shows a restriction enzyme map of the plasmid DNA, pNDPC 1 which is used in the examples of this invention.

Plasmid pNDPC 1 was constructed as follows: pUC 18 (Yarnisch-Perron, C. et al., Gene 33: 103(1985)) was partially digested by Cfr I or Ban I and then it was self ligated by T4-DNA ligase as shown in Figure 1. The selective markers were Ap and lac. pNDPC 1 with 2431 base pair was inserted into E.coli K12 Jm 109 by standard practiced procedures and the bacterium was cultured in a LB medium (1% trypton, 0.5% yeast extract, 0.5% NaCl). The plasmid was then purified by regular procedures.

The pNDPC 1 was then cut with restriction enzyme Cfrl or Ban I in an aqueous solution containing 10 mM of Tris HCL buffer pH 8.5 and containing 7 mM MgCl2, 20 mM NaCl and 7 mM 2-mercaptoethanol. The dsDNA obtained by using Cfr I contained 0.9 and 1.4 kbp while the dsDNA obtained by using Ban I contained 1.1 to 1.2 kbp.

## EXAMPLE 2

### Iodination of dsDNA from Example 1

In vitro iodination methods for labeling recombinant DNA molecules include nick translation, polynucleotide kinase, DNA polymerase large fragment (Klenow enzyme), T4-DNA polymerase and terminal deoxytransferase (TdT).

The nick translation method gives the highest specific activity but some of the labeled dsDNA seems to breakdown to ssDNA and is thus unsuitable for the present purposes. On the other hand, iodination methods using polynucleotide kinase, Klenow enzyme, T4-DNA polymerase, or TdT in which the terminals of the Dna are labeled, produce tracers of moderate activity compared to the nick translation method, but are devoid of ssDNA fragments. These methods are therefore the preferred iodination or labeling methods for this invention but should not be considered in any way restrictive. Other labeling methods will be obvious to those skilled in the art.

The dsDNA (1.1-1.2 kbp) obtained from Example 1, supra, was dissolved in an aqueous solution containing 50 mM Tris HCl buffer, pH 7.2 and containing 10 mM MgSO4, 1 mM dithiothreitol, 500 ug/ml bovine serum albumin. 1 mM of each dGTP, dATP and dTTP and 1 m Ci of 125I-cCTP and 25 units of T4 = DNA polymerase I large fragments (Klenow enzyme) were added and the reaction allowed to proceed at ambient temperature for 30 minutes.

The iodinated DNA was further purified by precipitation in 3.1 ml of 3 M sodium acetate pH 5.2 in 99.5 ethanol (0.1:3) at 080C for 30 minutes then centrifuged at 15,000 rpm. The supernatant was discarded and the precipitate dissolved in 1 ml of 10 mM Tris, 1 mM EDTA and 10 mM NaCl buffer pH 7.5 was reprecipitated with 3 M sodium acetate PH 5.2 and 99.5% ethanol as above. The supernatant was again discarded and the precipitate was washed with 70% ethanol and dried.

The iodinated DNA was resuspended in aqueous solution of 50 mM sodium borate, 15 mM EDTA and 0.01% sodium azide so that the final concentration was 0.1 uCi/ml. The resulting solution was used as the tracer with a specific activity of 0.3 uCi/pmol.

## EXAMPLE 3

### Assay Procedure for circulating Anti-DNA Anti-DNA Antibodies

Having prepared a well defined 125I-dsDNA label of given kbp and specific activity, capable of binding specifically to circulating anti-DNA immunoglobulins forms the basis of a sound method for measuring anti-DNA antibody titres in serum samples.

The following radioassay method is the preferred embodiment of the present invention but is by no means restrictive:

Anti-DNA calibrators were prepared from a serum of a patient suffering from SLE and diluted serially in normal human serum devoid of any anti-DNA antibodies. The calibrators were referenced against another radioassay produced by Amersham Corporation, Amersham England. The calibrators were assigned arbitrary units/ml (U/ml).

The 125-I-labeled dsDNA was diluted as shown above to give 75,000 cpm per 200 ul volume.

25 ul of each calibrator and sample are pipeted in 12 x 75 mm test tubes to which 200 ul of 125-I-dsDNA tracer was added. After incubating for 2 hours at 37C, 1.0 ml of 50% solution of aqueous ammonium sulfate was added to separate the immunoglobulin bound tracer from the free unbound tracer. After centrifugation at 2000 xg at 15 minutes the bound fraction was separated from the free fraction by decanting all tubes. The precipitate was counted and the patient sample concentrations were read off the calibration curve where the bound counts in the patient sample are directly proportional to the units of activity of anti-DNA titres.

EXAMPLE 4

Effect of Complement Clq

Ten normal samples were assayed by the method of this invention before and after heat treatment at 56C for 30 minutes to inactivate complement, Clq, using two tracers. Tracer 1 was the 125-I-dsDNA from Example 2, supra and is the preferred embodiment of this invention. Tracer 2 was an 125-I-ssDNA prepared as follows: the recombinant dsDNA (1.1 to 1.2 kbp) concentrate from Example 1, supra, was heated at 95C for one minute, cooled in ice and allowed to reach ambient temperature prior to iodination with 125-I as described in Example 2, supra. The resulting iodinated single stranded DNA (125-I-ssDNA) was then diluted in the tracer buffer and used in the assay.

The result of this experiment is summarized below:

| Tracer: | 1. 125I-dsDNA | 2. 125I-ssDNA |
|---|---|---|
| | B/T | |
| | Mean ± SD | |
| Samples (N=10) | | |
| Untreated | 4.5 ± 0.3 | 36.7 ± 2.2 |
| Treated, 56C 30 min | 4.4 ± 0.3 | 21.2 ± 6.7 |

The results clearly demonstrate that the double stranded DNA tracer, tracer 1, 125-I-dsDNA, is unaffected by the presence of complement in the patient sera. On the other hand, the single stranded DNA tracer, tracer 2, 125-I- ssDNA is grossly affected by complement in patient sera. In addition, even after inactivation of complement the 125-I-ssDNA gives falsely higher results mainly due to higher non-specific binding of the tracer to other circulating proteins.

EXAMPLE 5

Expected Values and Interpretation of Results

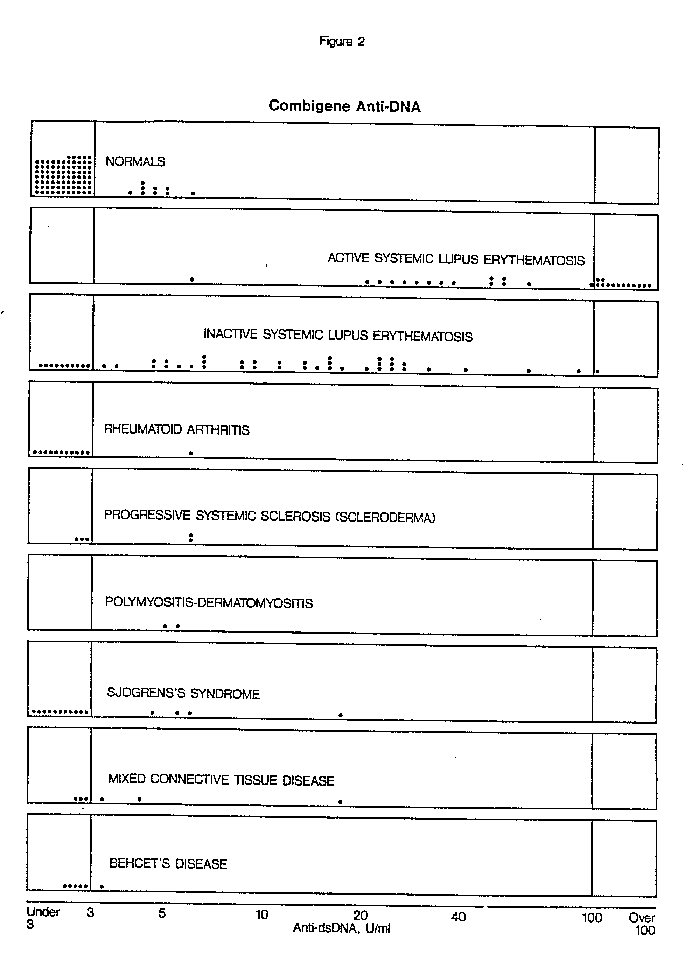

A total of 213 serum samples from normal individuals, patients with active and inactive systemic lupus erythematosus (SLE), and patients with various other connective tissue disorders were assayed by the method described in Example 3, supra. The results are presented graphically in figure 2.

0 276 984

| Condition | n |
|---|---|
| Normal Adults | 91 |
| Active SLE | 28 |
| Inactive SLE | 48 |
| Rheumatoid Arthritis | 12 |
| Scleroderma | 5 |
| Polymyositis-Dermatomyositis | 2 |
| Sjogren's Syndrome | 15 |
| Mixed Connective tissue Disease | 6 |
| Behcet's Disease | 6 |

All 91 normal individuals tested had anti-DNA results below 6 U/ml, as measured by the method of this invention, while all but one of the 28 patients with active SLE (96.4%) had results of 20 U/ml or higher. The one patient with active SLE had an anti-DNA level of 5.7 U/ml. In addition, 30 of the 48 patients with inactive SLE (62.5%) had results above 6 U/ml.

Of the 46 patients with connective tissue disorders other than SLE, only tow (4.4%) had anti-DNA levels above 6 U/ml.

A decision level set at approximately the upper limit of normal would thus maximize sensitivity for active SLE in this population, while minimizing the number of positive results in patients with disorders other than SLE.

Having fully described the invention it is intended that it be limited only by the lawful scope of the appended claims.

**Claims**

1. A method for measuring anti-DNA antibody titres in biological fluids by an immunological method using a dsDNA label free of ssDNA fragments, wherein the dsDNA label is prepared by recombinant DNA techniques and having a constant length of a well defined kbp and a given specific activity and that such labeled dsDNA be used as the tracer in said immunological method.

2. A method for measuring anti-DNA antibody titres in biological fluids according to claim 1 wherein such labeled dsDNA has a constant length of 1.1 to 1.2 kbp but may vary from about 0.2 to 3.0 kbp.

3. A method for measuring anti-DNA antibody titres in biological fluids according to claim 1 wherein said dsDNA labeled is labeled only at the terminals thereof by using an enzyme such as DNA polymerase large fragments (Klenow enzyme), polynucleotide kinase, T4-DNA polymerase, or terminal deoxytransferase.

4. A method for measuring anti-DNA antibody titres in biological fluids according to claim 1 wherein said dsDNA is labeled with a radioactive substance, an enzyme, a fluorescent substance, a chemiluminescent or bioluminescent substance.

5. An assay kit for measuring anti-DNA antibody titres in biological fluids by an immunological method using dsDNA label free of ssDNA fragments, wherein the dsDNA label is prepared by recombinant DNA techniques and having a constant length of a well defined kbp and a given specific activity and that such labeled dsDNA be used as the tractor in said immunological method.

6. An assay kit for measuring anti-DNA antibody titres in biological fluids according to claim 5 wherein such labeled dsDNA has a constant length of 1.1 to 1.2 kbp but may vary from about 0.2 to 3.0 kbp.

7. An assay kit for measuring anti-DNA antibody titres in biological fluids according to claim 5 wherein said dsDNA label is labeled only at the terminals thereof by using an enzyme sudh as DNA polymerase large fragments (Klenow enzyme), polynucleotide kinase, T4-DNA polymerase, or terminal deoxytransferase.

8. An assay kit for measuring anti-DNA antibody titres in biological fluids according to claim 5 wherein the label is a radioactive substance, an enzyme, a fluorescent substance, a chemiluminescent or bioluminescent substance.

9. An assay kit for use in determining anti-DNA antibody titres in a biological sample comprising a labelled double stranded DNA which is substantially free of single stranded DNA and has been prepared

5

by a recombinant DNA technique.

10. Use of a labelled double stranded recombinant DNA substantially free of single stranded DNA in an assay for anti-DNA autoantibodies.

Figure 1

- ▼ CfrI site
- ▽ Ban I site

0276984

Figure 2

**Combigene Anti-DNA**

NORMALS

ACTIVE SYSTEMIC LUPUS ERYTHEMATOSIS

INACTIVE SYSTEMIC LUPUS ERYTHEMATOSIS

RHEUMATOID ARTHRITIS

PROGRESSIVE SYSTEMIC SCLEROSIS (SCLERODERMA)

POLYMYOSITIS-DERMATOMYOSITIS

SJOGRENS'S SYNDROME

MIXED CONNECTIVE TISSUE DISEASE

BEHCET'S DISEASE

| Under 3 | 3 | 5 | 10 | 20 | 40 | 100 | Over 100 |

Anti-dsDNA, U/ml